Europäisches Patentamt

European Patent Office

Office européen des brevets

(19)

(11) Publication number: **0 466 268 A1**

## EUROPEAN PATENT APPLICATION

(21) Application number: **91201771.2**

(22) Date of filing: **05.07.91**

(51) Int. Cl.5: **B01J 27/22**, C07C 1/04

(30) Priority: **10.07.90 GB 9015193**

(43) Date of publication of application:
**15.01.92 Bulletin  92/03**

(84) Designated Contracting States:
**AT BE CH DE DK ES FR GB GR IT LI LU NL SE**

(71) Applicant: SHELL INTERNATIONALE
RESEARCH MAATSCHAPPIJ B.V.
Carel van Bylandtlaan 30
NL-2596 HR Den Haag(NL)

(72) Inventor: Geus, John Wilhelm
Croesestraat 77A
NL-3522 AD Utrecht(NL)
Inventor: Boellaard, Eliza
Croesestraat 77A
NL-3522 AD Utrecht(NL)

(54) **Catalysts and catalysts precursors suitable for hydrocarbon synthesis.**

(57) Catalysts and/or catalyst precursors comprise alloys or alloy particles of at least two metals chosen from the groups VIb, VIIb and VIII of the Periodic Table of Elements, the alloy having a negative heat of formation (with respect to the individual metals) and forming upon contacting with synthesis gas ($H_2$/CO ratio 2) at a temperature of 250 °C and under a pressure of 1 bar for a period of three hours a total amount of metal in the form of carbide of between 1 and 20 mol per cent based on the total amount of metal, while at least 50 per cent of the metal atoms present in the free surface area of the alloy have been converted into carbide. The catalysts and/or catalyst precursors are of use in the synthesis of hydrocarbons from synthesis gas.

EP 0 466 268 A1

The present invention relates to catalysts and/or catalyst precursors comprising alloys or alloy particles of at least two metals chosen from the groups VIb, VIIb and VIII of the Periodic Table of Elements, to a process for the preparation of these catalysts and/or catalyst precursors from complex cyanides and to the use of the catalysts and/or catalyst precursors in the synthesis of hydrocarbons from synthesis gas.

The preparation of hydrocarbons from gaseous mixtures comprising hydrogen and carbon monoxide by contacting these mixtures with a suitable catalyst at elevated temperature and pressure is known in the literature as the Fischer-Tropsch synthesis.

Catalysts often used for this purpose comprise one or more metals from the groups VIb, VIIb and VIII of the Periodic Table, especially from the iron group of group VIII, that is iron, nickel and cobalt, and ruthenium, supported on a carrier, optionally in combination with one or more metal oxides and/or other metals as promoters. The metal oxide promoters are usually chosen from groups Ia, IIa, IIIb, IVb, and/or Vb of the Periodic Table as well as from the lanthanides and/or actinides. The metal promoter may be selected from the groups VIIb and/or VIII of the Periodic Table.

Very suitable Fischer-Tropsch catalysts, especially catalysts containing iron and nickel on a carrier, especially an alumina carrier, have been described in the literature. Reference is made, for example, to Ullmanns Encyklopaedie der technischen Chemie, 3rd edition, Band 9, page 684.

The Fischer-Tropsch catalysts described above are usually prepared by combining a carrier and one or more suitable metal compounds, for example by precipitating the metals, either separately or together, on the carrier from one or more solutions, or by impregnating the carrier with compounds of the metals dissolved in a liquid in one or more steps. Alternatively, kneading techniques may be applied in the catalyst preparation. In all preparation procedures, solvent is usually removed by evaporation from the products obtained, followed by calcination of the dried products. Thereafter, the calcined product is activated prior to use, usually by reduction with a hydrogen containing gas.

In view of the growing demand for synthetic fuels and other valuable compounds which can be made by the Fischer-Tropsch process, there is an urgent need for new catalysts showing a high activity in combination with a high selectivity and a good stability.

It has now been found that very active Fischer-Tropsch catalysts which show a very good stability over long periods of time can be prepared by decomposition of complex cyanide loaded carriers under oxidative conditions, followed by activation with a hydrogen containing gas. This high level of stability is exhibited when the complex cyanides are derived from at least two metals chosen from the groups VIb, VIIb and VIII of the Periodic Table of Elements, and provided that the alloy of the two or more metals obtained after the reduction treatment has a negative heat of formation (with respect to the individual metals) and forms upon contacting with synthesis gas ($H_2$/CO ratio 2) at a temperature of 250 °C and a pressure of 1 bar for a period of three hours, a total amount of metal in the form of carbide of between 1 and 20 mol per cent based on the total amount of metal, while at least 50 per cent of the metal atoms present in the free surface area of the alloy have been converted into carbide.

The present application therefore relates to catalysts and/or catalyst precursors comprising alloys or alloy particles of at least two metals chosen from the groups VIb, VIIb and VIII of the Periodic Table of Elements, the alloy having a negative heat of formation (with respect to the individual metals) and forming upon contact with synthesis gas ($H_2$/CO ratio 2) at a temperature of 250 °C and under a pressure of 1 bar for a period of three hours, a total amount of metal in the form of carbide of between 1 and 20 mol per cent based on the total amount of metal, while at least 50 per cent of the metal atoms present in the free surface area of the alloy have been converted into carbide.

The alloy particles to be used in the process of the present invention preferably have substantially the same chemical composition, which means that every alloy particle should have the same relative amounts of the metals which make up the particle. It will be appreciated that catalysts and/or catalyst precursors comprising more than one alloy, each alloy having the composition and features as described above, and in which the particles of each alloy have substantially the same chemical composition, are also within the scope of the present invention.

The preferred metals for inclusion in the catalysts of the present invention are selected from group VIII of the Periodic Table of Elements, in particular iron, cobalt and nickel. Preferably iron and nickel are used. Two metals may be used alone, but also three, or even more metals, may be used. It will be appreciated that, once a certain combination of metals has been found, additional metals may be added to the alloy by way of optimization to find the best alloy composition with respect to the synthesis of hydrocarbons from synthesis gas. Optionally, one or more promoters may be added, for example the promoters as hereinbefore described.

When iron and nickel are present in the alloy, the amount of iron in the iron-nickel alloy is suitably from 30 to 55 mol %, preferably about 40 mol %, the amount of nickel in the alloy is suitably from 45 to 70 mol %, preferably about 60 mol %.

The catalysts according to the present invention may also comprise a carrier. Refractory oxides as well as zeolites and mixtures thereof can be used as carrier. Examples of suitable carriers are alumina, silica-alumina, titania, magnesia or zirconia or mixtures thereof. Alumina is a particularly preferred carrier material. Crystalline (metallo)-silicates may also be used, especially crystalline alumina silicates. It may be preferred to subject the refractory oxides to an activating treatment prior to the incorporation of the complex cyanides therein.

Typically, the amount of carrier varies from 5 to 95% by weight of the total catalyst, especially from 15 to 85% by weight. The carrier to be used suitably has a pore volume ($H_2O$) on a dry basis of from 0.1 to 1.0 ml/g, preferably from 0.3 to 0.7 ml/g. The surface area is suitably from 10 to 600 $m^2/g$, preferably from 20 to 450 $m^2/g$, especially from 50 to 300 $m^2/g$. The particle diameter is suitably from 0.1 to 12 mm, preferably from 0.5 to 5 mm, for catalyst particles to be used in fixed bed reactors, and from 0.5 to 25 micron, preferably from 1 to 10 micron, for catalyst particles to be used in slurry reactors. It will be appreciated that the catalyst particles may be mixed with other particles, for example inert diluent particles. Also particles comprising one or more other catalytic activities may be used for mixing purposes.

The alloy particles to be used in the catalyst of the present invention should preferably have substantially the same chemical composition, that is, each alloy particle should contain the same relative amounts of the metals which compose the alloy. In this respect it is observed that in general the prior art processes for the production of catalysts comprising two or more metals do not result in the production of alloy particles which have substantially the same composition. The average size of the alloy particles is suitably from 2 to 100 nm, preferably from 5 to 30 nm.

The alloy to be used in the catalyst particles of the present application should have a negative heat of formation with respect to the starting metals themselves, that is heat should be evolved by forming the alloy from the composing metals. It will be appreciated that literature data are available describing heats of formation of binary alloys from the metals which can be used for the catalysts of the present invention. In this respect reference is made, for example, to R. Hultgren, P.D. Desai, D.T. Hawkins, M. Gleiser and K.K. Kelley, Selected values of the thermodynamic properties of binary alloys, Amer. Soc. for Metals, Metals Parc, OH, 1973. The negative heat of formation is preferably from 0.1 to 10 kJ/mol, more preferably from 0.4 to 5 kJ/mol.

The alloy to be used for the catalysts of the present invention should form upon contacting with synthesis gas ($H_2$/CO ratio 2) at a temperature of 250 °C and under a pressure of 1 bar for a period of three hours a total amount of metal in the form of carbide of from 1 to 20 mol % based on the total amount of metal, while at least 50% of the metal atoms present in the free surface area of the alloy have been converted into carbide. The total amount of metal in the form of carbide can be measured according to standard testing methods, for example as described by J.W. Niemantsverdriet, A.M. van der Kraan, W.L. van Dijk and H.S. van der Baan, J. Phys. Chem., 84, 3363 (1980). The total amount of metal in the form of carbide is preferably from 5 to 15 mol %. The amount of metal atoms present in the free surface area which have been converted into carbide upon contact with synthesis gas in relation to the total amount of metal atoms in the surface area can be established by temperature programmed surface reaction, for example as described by J.G. McCarthy et al in "Coke formation on metal surfaces" (L.F. Albright and R.T.K. Baker Eds.), A.C.S. Symposium Series 202, page 253 (Am. Chem. Soc. Washington D.C., 1982).

The free surface area of the alloys, that is the surface area which is freely accessible from the gas phase, can be determined by known methods, for example the methods described by Topsoe and/or Emmett in which the extent of chemisorption of carbon monoxide can be determined (H. Topsoe, N. Topsoe, N. Bohlbro and J.A. Dumesic in "Proceedings of the 7th International Congress on Catalysis, Tokyo, 1980" (T. Seiyama and K. Tanabe Eds.) p. 247, Elseviers Scientific Publishing Company, Amsterdam (1981); P.H. Emmet and S. Brunauer, J. Am. Chem. Soc., 59, (1937) 310, and S. Brunauer and P.H. Emmett, J. Am. Chem. Soc., 62, (1940) 1732).

Without wishing to be bound to any particular theory it may be observed that the surprising activity and performance of the catalysts as hereinbefore described may be related to the fact that the alloy shows a high rate of formation of surface carbide which is regarded as the starting species for the Fischer-Tropsch synthesis, while bulk carbide formation, which results in a decrease of the activity, does not occur to any substantial degree. Thus, a high activity catalyst is obtained which does not show any signs of deactivation over long reaction periods. In this respect it is observed that, for example, iron per se shows a relatively high rate of bulk carbide formation, and therefore is not suitable as a Fischer-Tropsch catalyst for long reaction periods. Iron/copper alloys show positive heats of formation, resulting in separation of the composing metals, in turn resulting in lower activity Fischer-Tropsch catalysts.

The catalysts according to the present invention very suitably may be prepared by decomposition of a complex cyanide, followed by an activation procedure. The present invention therefore also provides a process for the preparation of catalysts or catalyst precursors as described above, wherein a complex cyanide of the general formula $M_1M_2(CN)_{p-x}(Y)_x$, in which $M_1$ represents a cationic moiety and $M_2$ forms part of the anionic moiety, and $M_1$ and $M_2$ each represent a metal chosen from the groups VIb, VIIb or VIII of the Periodic Table, CN represents a cyanide moiety, Y represents one or more ligands, p is a number ranging from 2 to 8, x is a number ranging from 0 to 4 and p/x is at least 1 when x>0, present on a carrier is subjected to a decomposition treatment under oxidative conditions, optionally followed by a reduction treatment.

For an extensive description of the above indicated process reference is made to British patent application No. 9005964.3.

It should be noted that complex cyanides can be used as starting materials in the preparation of supported catalysts by introducing an appropriate cyanide into a support, or, alternatively, by precipitating a complex cyanide onto a support from a metal salt and an appropriate cyanide, followed by activating the cyanide thus introduced. For example, in U.S. patent specification 4,186,112 a process is described for reducing carbon monoxide by means of hydrogen using supported catalysts prepared by precipitating a polymetal salt of a hydrocyanic acid which is subjected to a so-called forming step, after separating and drying the precipitated salt. It is reported that forming takes place upon thermally decomposing the salt in contact with hydrogen or a mixture of hydrogen and carbon monoxide. It is also possible to carry out the thermal decomposition under vacuum.

It is further known (J. Catal. (71), 111 - 118 (1981)) to produce finely dispersed metals in zeolites by reacting a metal-exchanged zeolite and an anionic, metal-containing coordination compound, specifically a water-soluble, metal cyanide complex followed by subsequent reduction with hydrogen at a temperature of 400 °C.

Typically, the decomposition treatment under oxidative conditions is carried out in an environment containing at least 0.5% by volume of an oxidising agent. It is also possible, and in fact preferred, to use an environment containing a larger amount of oxidising agent.

Examples of oxidising agents comprise oxygen, ozone and hydroperoxides. The environment containing the oxidising agent normally comprises an inert medium, such as an inert gaseous compound, for example nitrogen, argon or helium. Preferably, air is used to perform the decomposition treatment in accordance with the present invention. The air may be diluted, if desired, by one or more inert gases, for example nitrogen.

The decomposition treatment under oxidative conditions is typically carried out at a temperature of at least 200 °C. Preference is given to a temperature of from 250 °C to 450 °C. The optimum temperature to be applied depends to some extent upon the type and number of metal moieties present in the complex cyanide containing carrier.

The complex cyanide containing carrier is typically subjected to the decomposition treatment under oxidative conditions of the present invention when present in a closed vessel with venting facilities, such as an autoclave or a rotating kiln.

Complex cyanides present on carriers which can be subjected to the decomposition treatment in accordance with the present invention can be represented by the general formula $M_1M_2(CN)_{p-x}(Y)_x$ wherein $M_1$ and $M_2$ are as defined hereinbefore. It is possible that some interchange may take place between moieties $M_1$ and $M_2$ during the formation of the complex cyanides. It is also possible that part of the $M_1$ moiety is replaced by another moiety such as $H^+$, $NH_4 +$ or a quaternary ammonium ion, for example by means of an ion-exchange mechanism. When $M_1$ represents a quaternary ammonium ion preference is given to the lower quaternary alkyl ammonium ions such as tetramethyl, tetraethyl and dimethyl diethyl ammonium ions. The presence of quaternary ammonium ions may have an advantageous effect when non-aqueous impregnation methods are envisaged.

The expression "a cyanide moiety" as used throughout the present specification includes, apart from the cyano group (the proper cyanide group), the isocyano group, the thiocyano group, the isothiocyano group, the cyanato group and the isocyanato group. Preference is given to the presence of the proper cyanide group in the complex cyanide structure.

Depending on the coordinative preferences of the (various) metal(s) present in the complex cyanide, the number for p can range from 2 to 8. Also the number of Y moieties present has a bearing on the value for p. Preferably, p represents an even number, in particular 4, 6 or 8. For complex cyanides having $M_2$ representing palladium, p is equal to 4, for complex cyanides having $M_2$ representing iron, p is equal to 6 and for complex cyanides having $M_2$ representing molybdenum, p is equal to 8.

The Y moiety typically represents one or more of NO, CO, $NH_3$, $NO^+$ or $NO_2^-$. Preferably Y represents one or more NO moieties. Up to four ligands Y may be present in the complex cyanides, provided that the ratio p/x is at least 1 when x > 0. Preference is given to complex cyanides containing not more than 2 and in particular no ligands Y.

Without wishing to be bound to any particular theory it should be noted that the surprising activity and performance of catalysts produced in accordance with the decomposition treatment under oxidative conditions may be related to the observation that water present in the complex cyanides when precipitated on the appropriate carrier is preferentially removed prior to the decomposition treatment according to the present invention. When the cyanide group is decomposed in the substantial absence of water, a decomposition mechanism seems to apply which substantially prevents the unwanted interaction between the metal(s) remaining on the carrier and the sites on the carrier normally exposed to undergo metal/site interaction.

The process according to the present invention is suitably carried out by using a complex cyanide wherein $M_1$ and $M_2$ each represent a Group VIb, VIIb or VIII metal moiety, optionally partly replaced by moieties, such as $H^+$ and $NH_4 +$, CN represents cyanide, p represents 4, 6 or 8 and Y represents a NO group when x is not zero.

Preferably, the process according to the present invention is carried out by using a complex cyanide wherein $M_1$ and $M_2$ each represent a Group VIII metal moiety, p represents 4, 6 or 8 and x is zero. In particular, use is most preferably made of a complex cyanide wherein $M_1$ and $M_2$ each represent a Fe, Co or Ni moiety.

As regards the various Groups of the Periodic Table of the Elements reference is made to the Handbook of Chemistry and Physics, 64th Edition, 1983 published by the Chemical Rubber Company.

Examples of complex cyanides which can be used (after having been incorporated on a carrier) as starting materials in the decomposition treatment in accordance with the present invention include: $Ni_2Fe(II)(CN)_6$, $Ni_3(Fe(III)(CN)_6)_2$, $NiFe(CN)_5NO$, $FePd(CN)_4$, $Co_3[Fe(III)(CN)_6]_2$, $Co_2Mo(CN)_8$, $CoFe(CN)_5NO$ and $Mn_2Fe(II)(CN)_6$, Normally, the complex cyanides will contain water of hydration.

The complex cyanides can be suitably incorporated on the appropriate carrier by in situ formation, for example by reaction of one or more metal compounds containing $M_1$ moieties, in particular one or more salts containing $M_1$ moieties, and a cyanide containing a $M_2$ moiety.

Preferably, the complex cyanides are incorporated on the carrier by treating the appropriate carrier with one or more cyanides containing a $M_2$ moiety followed by drying and subjecting the carrier containing the cyanide to impregnation with one or more compounds containing $M_1$ moieties, in particular one or more salts containing $M_1$ moieties, so as to form the complex cyanide. It is also preferred to incorporate the complex cyanides in the appropriate carrier by treating the appropriate carrier with one or more compounds containing $M_1$ moieties, in particular one or more salts containing $M_1$ moieties, followed by drying and subjecting the carrier containing the $M_1$ moiety to impregnation with one or more cyanides containing a $M_2$ moiety so as to form the complex cyanide.

It is also possible to treat an appropriate carrier with a soluble complex cyanide and dry the thus treated carrier to produce the complex cyanide(s)-containing carrier to be subjected to a decomposition treatment under oxidative conditions according to the present invention.

It is further possible to use the so-called incipient wetness impregnation method. Using this method gives the opportunity to introduce either one or more salts containing one or more $M_1$ moieties or the cyanide containing a $M_2$ moiety on the carrier.

When salts containing one or more $M_1$ metal moieties and cyanide containing a $M_2$ moiety have been introduced onto the carrier, resulting in the formation of the appropriate complex cyanide(s), the metal moieties $M_1$ and $M_2$ are still in non-zero valencies. By subjecting the complex cyanides to a decomposition treatment under oxidative conditions it will be clear that the cyanide moieties will be substantially destroyed, which will leave the $M_1$ and $M_2$ metal moieties substantially in the appropriate oxidic form.

In the event that it is desired, for example for catalytic purposes, to have the metal moieties present in substantial amounts in the zero valency state, it will be necessary to subject the complex cyanides to a reducing treatment. Such a treatment, which will normally activate or increase the catalytic behaviour of the catalysts, may be carried out in the presence of hydrogen at a temperature up to 500 °C and at a pressure of up to 10 MPa. Preferably, the reducing treatment is carried out at a temperature in the range of from 50 °C to 300 °C.

The complex cyanides present on a carrier which have been subjected to a decomposition treatment in accordance with the present invention can be used, either as such or after a reducing treatment as discussed hereinbefore, as catalyst precursors or as catalysts in the synthesis of hydrocarbons from synthesis gas. Without wishing to be bound to any particular theory it is thought that the attractive catalytic properties of the catalysts obtainable from complex cyanides as defined hereinbefore (after at least the decomposition treatment under oxidative conditions) are related to the very effective dispersion of the appropriate metal moieties throughout the system.

The invention further relates to a process for the preparation of hydrocarbons and/or oxygenates from a mixture of carbon monoxide and hydrogen by contacting the mixture with a catalyst as described hereinbefore. The conversion of the mixture of hydrogen and carbon monoxide may be carried out at a temperature of from 200 to 350 °C, and a pressure of from 5 to 100 bar, preferably from 10 to 30 bar, and a space velocity of from 20 to 20000 $m^3$ (S.T.P.) gaseous feed/$m^3$ reaction zone/hour, preferably from 500 to 5000 $m^3$ (S.T.P.) gaseous feed/$m_3$ reaction zone/hour.

The hydrogen and carbon monoxide containing feed to be converted into hydrocarbons by using a catalyst according to the present invention preferably has an $H_2$/CO molar ratio of from 0.5 to 2.5, preferably from 1.5 to 2.2. It is observed that unconverted hydrogen and carbon monoxide may be recirculated over the catalyst bed in such a way that the catalyst is contacted with a synthesis gas feed having a substantially lower $H_2$/CO ratio than that of the feed synthesis gas. Thus, the selectivity to longer hydrocarbon chains may be improved.

Normally synthesis gas is used as feed gas. Synthesis gas contains as major components hydrogen and carbon monoxide. In addition it may contain small amounts of carbon dioxide, water, nitrogen, argon and minor amounts of compounds having 1 to 4 carbon atoms per molecule, such as methane, ethene etc. Further, methanol may be used as feed gas, either as such or in combination with hydrogen and/or carbon monoxide.

The synthesis gas may be prepared by any manner known in the art, for example by means of steam/oxygen gasification of hydrocarbonaceous material such as brown coal, anthracite, coke, crude mineral oil and (heavy) fractions thereof, oil recovered from tar sand and bitumous shale. Alternatively, steam methane reforming and/or catalytic partial oxidation of a hydrocarbonaceous material with an oxygen-containing gas may be used to produce synthesis gas.

It will be appreciated that the invention also relates to the hydrocarbon products obtained with the catalysts as described hereinbefore.

The invention is further illustrated by the following examples.

EXAMPLE 1

2.31 grammes of $K_4Fe(CN)_6.3H_2O$ were dissolved in 100 ml of water and slowly injected into a solution of 2.64 grammes of $Cu(NO_3).3H_2O$ in 1000 ml water, in which 4.00 grammes of $Al_2O_3$ - (Degussa Aluminium Oxid C) had been suspended. The pH of the vigorously stirred solution was 5.0 and the temperature was 22 °C. The resulting precipitate was suction-filtered, washed with 1000 ml and vacuum dried. The product was pelletised, crushed and a fraction of from 0.43 to 0.71 mm of the $Cu_2$/Fe-complex cyanide was obtained.

The alumina - $Cu_2$/Fe-complex cyanide was oxidised using an oxidation gas comprising 99% by volume of argon and 1% by volume of oxygen at a temperature of 245 °C.

EXAMPLE 2

The experiment of Example 1 was repeated using 2.52 grammes of $K_4Fe(CN)_6.3H_2O$ and 2.37 grammes of $FeCl_2.4H_2O$. The corresponding $Fe_2$/Fe complex was obtained. The oxidation was carried out at 290 °C.

EXAMPLE 3

The experiment of Example 1 was repeated using 2.44 grammes of $K_4Fe(CN)_6.3H_2O$ and 3.36 grammes of $Ni(NO_3)_2.6H_2O$. The corresponding $Ni_2$/Fe complex was obtained. The oxidation was carried out at 305 °C.

EXAMPLE 4

The experiment of Example 1 was repeated using 2.29 grammes of $K_3Fe(CN)_6$ and 3.03 grammes of $Ni(NO_3)_2.6H_2O$. The corresponding $Ni_3$/$Fe_2$ complex was obtained. The oxidation was carried out at 310 °C.

EXAMPLE 5

The experiment of Example 1 was repeated using 2.60 grammes of $Na_2Fe(CN)_5NO.2H_2O$ and 2.54 grammes of $Ni(NO_3)_2.6H_2O$. The corresponding Ni/Fe complex was obtained. The oxidation was carried out at 335 °C.

EXAMPLE 6

The experiment of Example 1 was repeated using 2.43 grammes of $K_4Fe(CN)_6.3H_2O$ and 3.35 grammes of $Co(NO_3)_2.6H_2O$. The corresponding $Co_2$/Fe complex was obtained. The oxidation was carried out at 290 °C.

EXAMPLE 7

The experiment of Example 1 was repeated using 2.28 grammes of $K_3Fe(CN)_6$ and 3.03 grammes of $Co(NO_3)_2.6H_2O$. The corresponding $Co_3$/$Fe_2$ complex was obtained. The oxidation was carried out at 325 °C.

EXAMPLE 8

The experiment of Example 1 was repeated using 2.60 grammes of $Na_2Fe(CN)_5NO.2H_2O$ and 2.54 grammes of $Co(NO_3)_2.6H_2O$. The correspond-

ing Co/Fe complex was obtained. The oxidation was carried out at 330 °C.

## EXAMPLE 9

Catalyst testing

The catalysts prepared as described in Examples 1 to 8 were tested in the synthesis of hydrocarbons from synthesis gas in a tubular reactor comprising a fixed catalyst bed with a bulk volume of 1 ml.

Prior to testing, the catalysts were reduced with hydrogen under the following conditions: pressure: 0.1 MPa; temperature: programmed heating procedure during 64 hours from 100 °C to 450 °C (except the catalysts of example 1: 100 °C to 275 °C) reduction gas: argon/hydrogen 9:1.

The conditions during the conversion of synthesis gas were: pressure: 0.1 MPa; temperature 250 °C; $H_2/CO$ ratio: 2; GHSV: 60 Nl/l/min.

The catalysts prepared in Examples 3 to 5 showed a high initial activity, and a good steady state activity in hydrocarbon synthesis. The catalyst of Example 1 showed a low initial activity and a low steady state activity, while the catalyst of Example 2 showed a large production of carbon, together with a quick decrease of activity. The catalysts of Examples 6 to 8 showed a low activity.

## Claims

1. Catalysts and/or catalyst precursors comprising alloys or alloy particles of at least two metals chosen from the groups VIb, VIIb and VIII of the Periodic Table of Elements, the alloy having a negative heat of formation (with respect to the individual metals) and forming upon contacting with synthesis gas ($H_2/CO$ ratio 2) at a temperature of 250 °C and under a pressure of 1 bar for a period of three hours a total amount of metal in the form of carbide of between 1 and 20 mol per cent based on the total amount of metal, while at least 50 per cent of the metal atoms present in the free surface area of the alloy have been converted into carbide.

2. Catalysts and/or catalyst precursors according to claim 1, characterized in that the metals are chosen from iron, cobalt and nickel, in particular iron and nickel.

3. Catalysts and/or catalyst precursors according to claim 2 in which metals are iron and nickel, characterized in that the amount of nickel is from 45 to 70 mol % based on the amount of alloy, and the amount of iron is from 30 to 55 mol % based on the amount of alloy.

4. Catalysts and/or catalyst precursors according to claim 3, characterized in that the amount of nickel is about 60 mol % and the amount of iron is about 40 mol %.

5. Catalysts and/or catalyst precursors according to any one of claims 1 to 4, characterized in that the catalyst or catalysts precursor comprises a carrier, in particular a refractory oxide, preferably alumina.

6. Catalysts and/or catalyst precursors according to any one of claims 1 to 5, characterized in that the negative heat of formation is from 0.1 to 10 kJ/mol, preferably from 0.4 to 5 kJ/mol.

7. Catalysts and/or catalyst precursors according to any one of claims 1 to 6, characterized in that the alloy upon contacting with synthesis gas forms a total amount of carbide of from 5 to 15 mol %, and from 60 to 90% surface carbide.

8. Catalysts and/or catalyst precursors according to any one of claims 1 to 7, characterized in that the average particle size of the alloy particles is from 2 to 100 nm.

9. Process for the preparation of a catalyst and/or a catalyst precursor according to any one of claims 1 to 8, wherein a complex cyanide of the general formula $M_1M_2CN)_{p-x}(Y)_x$ wherein $M_1$ represents a cationic moiety and $M_2$ forms part of the anionic moiety, and $M_1$ and $M_2$ each represent a metal chosen from the groups VIb, VIIb or VIII of the Periodic Table, CN represents a cyanide moiety, Y represents one or more ligands, p is a number ranging from 2-8, x is a number ranging from 0-4 and p/x is at least 1 when x>0, present on a carrier is subjected to a decomposition treatment under oxidative conditions, optionally followed by a reduction treatment.

10. Process according to claim 9, characterized in that the oxidative treatment is carried out at a temperature of at least 200 °C, preferably from 250 °C to 450 °C, in an environment containing at least 0.5% by volume of an oxidising agent, preferably oxygen.

11. Process according to either of claims 9 or 10, characterized in that $M_1$ and $M_2$ each represent a metal chosen from the iron group of the Periodic Table, p represents 4,6 or 8 and Y represents a NO group when x is not zero.

12. Process according to any of claims 9 to 11, characterized in that $M_1$ represents nickel, $M_2$ represents iron and p represents 6.

13. Process for the preparation of hydrocarbons and/or oxygenates from a mixture of carbon monoxide and hydrogen by contacting the mixture with a catalyst according to any one or more of claims 1 to 8 at elevated temperature and pressure.

European
Patent Office

EUROPEAN SEARCH
REPORT

Application Number

EP 91 20 1771

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | US-A-4 687 753 (FIATO) <br> – – – | | B 01 J 27/22 <br> C 07 C 1/04 |
| A,D | US-A-4 186 112 (VOGT) <br> – – – | | |
| A | US-A-2 601 121 (MATTOX) <br> – – – – – | | |

TECHNICAL FIELDS
SEARCHED (Int. Cl.5)

B 01 J
C 07 C

The present search report has been drawn up for all claims

| Place of search | Date of completion of search | Examiner |
|---|---|---|
| The Hague | 19 September 91 | MEERTENS J. |